Europäisches Patentamt

European Patent Office

Office européen des brevets

(11) Veröffentlichungsnummer : **0 331 954 B1**

(12) # EUROPÄISCHE PATENTSCHRIFT

(45) Veröffentlichungstag der Patentschrift :
**12.08.92 Patentblatt 92/33**

(51) Int. Cl.⁵ : **A61M 25/02**

(21) Anmeldenummer : **89102862.3**

(22) Anmeldetag : **18.02.89**

(54) **Vorrichtung zur Befestigung eines Katheters.**

(30) Priorität : **02.03.88 DE 8802756 U**

(43) Veröffentlichungstag der Anmeldung :
**13.09.89 Patentblatt 89/37**

(45) Bekanntmachung des Hinweises auf die
Patenterteilung :
**12.08.92 Patentblatt 92/33**

(84) Benannte Vertragsstaaten :
**AT BE CH DE ES FR GB GR IT LI LU NL SE**

(56) Entgegenhaltungen :
**EP-A- 0 247 571**
**DE-U- 7 801 811**
**DE-U- 8 204 827**
**US-A- 4 314 568**
**US-A- 4 632 670**

(73) Patentinhaber : **B. Braun Melsungen AG**
**Carl-Braun Strasse**
**W-3508 Melsungen (DE)**

(72) Erfinder : **Haindl, Hans, Dr.**
**Schoene Aussicht 4**
**W-3508 Melsungen (DE)**

(74) Vertreter : **Selting, Günther, Dipl.-Ing. et al**
**Patentanwälte von Kreisler, Selting, Werner**
**Deichmannhaus am Hauptbahnhof**
**W-5000 Köln 1 (DE)**

EP 0 331 954 B1

## Beschreibung

Die Erfindung bezieht sich auf eine Vorrichtung zur Befestigung eines transcutanen oder implantierten Katheters, bestehend aus einer Platte aus biegbarem Material, die mit einer endseitig offenen, axial geschlitzten Klemmrille für mit federelastischen Seitenflanken den Katheter versehen ist.

Eine sichere Fixierung eines Katheters, sei es ein Periduralkatheter oder ein Gefäßkatheter, muß möglichst direkt an der Punktionsstelle erfolgen. Auch implantierte Katheter müssen zur Vermeidung von Dislokationen an bestimmten Stellen fixiert werden. Zu diesem Zweck sind Fixationsvorrichtungen entwickelt worden, die sich grob in klebende und klemmende Vorrichtungen unterteilen lassen. Klebende Vorrichtungen verwenden haftfähige Folien, die den aus der Punktionsstelle austretenden Katheterteil direkt auf der Haut festkleben oder bei sandwichartiger Anordnung den Katheterteil zwischen sich haftend fixieren (DE-A-36 43 985 bzw, EP-A-0 247 571). Solche klebenden Vorrichtungen funktionieren z.B. bei Periduralkathetern nicht. Der Grund hierfür liegt teilweise in der schlechten Klebbarkeit der verwendeten Kathetermaterialien (z.B. Polyamid), teilweise aber auch darin, daß im unmittelbaren Wundbereich stark klebende Adhäsive nicht verwendet werden können. Für implantierte Katheter sind klebende Vorrichtungen gar nicht anwendbar.

Bei einer bekannten klemmenden Vorrichtung weist ein Clips zwei gelochte Klemmplatten mit Klammerelementen zur Einspannung des Katheters auf. Die Lochungen erlauben ein Festnähen des Clipses auf der Haut (DE-U-82 04 827). Die beiden Klemmplatten bestehen aus steifem Kunststoff, so daß der Clips starr und hart ist. Aus diesem Grunde kann er nicht als Implantat verwendet werden. Auch kann er nicht im Bereich des Rückens zur Anwendung kommen, da die Patienten auf den steifen Clipsen nicht liegen können. Die Steifheit des Clipses hat außerdem zur Folge, daß seine Fixierfähigkeit im wesentlichen auf Polyurethan-Katheter beschränkt ist, die eine gewisse materialbedingte Oberflächenklebrigkeit aufweisen. Polyamid-Katheter, z.B. Peridural-Katheter, werden infolge mangelnder Oberflächenhaftung in dem steifen Clips nicht zuverlässig festgehalten.

Ferner ist die klemmende Vorrichtung der eingangs erwähnten Art bekannt (DE-U-78 01 811). In diesem Falle erhält eine streifenförmige dünne Platte durch schlaufenartige Umlenkung mehrere parallele Klemmrillen, deren Durchmesser zur Erzielung einer Klemmwirkung kleiner sind als der Außendurchmesser des zu fixierenden Katheters. Der axiale Schlitz jeder Klemmrille verläuft in der ebenen Unterseite der Platte. Durch Biegung der Platte wird der axiale Schlitz jeder Klemmrille zum Einlegen von Katheterabschnitten aufgeweitet und in geradegerichtetem Zustand wird die Platte mit der Hautoberfläche zugewandter ebener Seitenfläche mittels Pflastern auf der Hautoberfläche festgeklebt. Als Implantat ist diese bekannte Befestigungsvorrichtung nicht nur wegen der problematischen Klebefixierung, sondern auch wegen der dünnen Ausbildung der Platte ungeeignet, die einer stützenden Auflagefläche für ihre ebene Seitenfläche bedarf, damit sie sich nicht so verbiegt, daß die Klemmrillen durch Aufweitung ihre Klemmwirkung für den Katheter verlieren.

Der Erfindung liegt die Aufgabe zugrunde, die Befestigungsvorrichtung nach DE-U-78 01 811 so zu verbessern, daß sie transcutane und implantierte Katheter beliebiger Beschaffenheit körperverträglich zuverlässig fixiert.

Diese Aufgabe wird erfindungsgemäß dadurch gelöst, daß die Platte einen von zwei vorsprungslosen Seitenflächen begrenzten weichflexiblen, knopfförmigen Plattenkörper aufweist, in den die Klemmrille eingelassen ist.

Der weichflexible, knopfförmige Plattenkörper, der vorzugsweise aus Polyurethan hergestellt ist, wird in der Mitte von einem Ende zum anderen von der Klemmrille durchquert, deren Höhe bis auf einen Grundsteg an ihrer geschlossenen Basis der Dicke des Plattenkörpers entspricht. Die Außenfläche des Grundsteges der Klemmrille liegt bündig in der einen Seitenfläche des Plattenkörpers, die vorzugsweise konvex gewölbt ist, und die axiale Schlitzöffnung der Klemmrille schließt bündig mit der anderen Seitenfläche des Plattenkörpers ab, die im wesentlichen eben sein kann. Durch Verbiegen des Plattenkörpers wird die Klemmrille geöffnet, so daß sich der Katheter einlegen läßt. Beim elastischen Zurückfedern des weichen Plattenkörpers üben die Seitenflanken der Klemmrille federelastisch einen leichten Druck auf den Katheter aus, der ihn mit dem Plattenkörper verbunden hält. Die weiche Flexibilität des knopfförmigen Plattenkörpers begründet seine Eignung als Befestigungsteil auch für Katheter ohne Oberflächenklebrigkeit, die wie z.B. Peridural-Katheter, aus Polyamid hergestellt sind. Der knopfförmige Plattenkörper läßt sich infolge seiner Vorsprungslosigkeit auf beiden Seiten und seiner Weichheit auch im Rückenbereich eines Patienten anbringen, ohne ihn beim Liegen zu behindern. Da bei ausreichender Dicke des Plattenkörpers und des Grundsteges der Klemmrille die inhärente Rückstellfähigkeit des weichflexiblen Materials zum Zusammenhalt der Klemmrille ausreicht, kann die Befestigungsvorrichtung auch für implantierte Katheter mit Erfolg angewendet werden. In diesem Falle kann eine Fixation durch Vernähen erfolgen. Auf der Hautoberfläche kann der Plattenkörper festgenäht oder festgeklebt werden.

Zur Erleichterung des Festnähens des Plattenkörpers auf der Haut oder im Gewebe ist der Plattenkörper auf beiden Längsseiten der Klemmrille mit durchgehenden Löchern versehen. Diese dienen außerdem der Verstärkung und Aufrechterhaltung des Klemmdruckes der Klemmrille durch Anbringung von zwei Fadenligaturen durch Lochpaare derart, daß diese die Klemmrille überspannen.

Der Plattenkörper ist zweckmäßigerweise in dem die gerade Klemmrille aufweisenden Mittelbereich verdickt ausgebildet, und es ist vorgesehen, daß die dem Axialschlitz der Klemmrille abgewandte Seitenfläche quer zur Längsachse der Klemmrille konvex gewölbt ist. Auf diese Weise wird eine erhöhte Klemmfähigkeit des Plattenkörpers in der Klemmrillenzone erreicht und seine zu der Klemmrille parallelen Außenränder sind abgeflacht, was für die Befestigung des Plattenkörpers auf der Haut günstig ist.

Die Klemmrille ist im Querschnitt vorzugsweise im wesentlichen kreisförmig. Sie kann alternativ etwa U-förmig sein. Im ersten Falle ist der Axialschlitz der Klemmrille zwischen zwei einander gegebenenfalls berührenden Lippen ausgebildet, während im zweiten Falle der Axialschlitz offener ist. Bei beiden Ausführungsformen ist vorteilhafterweise an wenigstens einem Ende der Klemmrille wenigstens ein zu der Dicke des Plattenkörpers quergerichteter Durchlaß für den abgewinkelten Katheter ausgebildet. Dieser kann ein Loch sein oder es kann sich um eine gegen das Plattenende offene Schlitzung handeln. Die Durchlässe machen es möglich, den in der Klemmrille gehaltenen Katheter rechtwinklig, d.h. senkrecht zur Ebene des Plattenkörpers, aus diesem herauszuführen. Dadurch kann der Plattenkörper erforderlichenfalls direkt über der Eintrittsstelle des Katheters in die Haut oder in ein darunterliegendes Gewebe fixiert werden. Die Abstützung an der Umlenkstelle wirkt dabei als Knickschutz für den Katheter.

Wenn eine Zusammenhaltung der Klemmrille durch Ligatur unerwünscht ist, kann eine erfindungsgemäße Zuhaltung für den Axialschlitz der Klemmrille verwendet werden. Diese kann aus einem den Axialschlitz der Klemmrille überspannenden Riegel mit Vorsprüngen bestehen, die in einige der Löcher des Plattenkörpers eingeschnappt sind. Der Riegel kann als Kunststoffbrücke mit vier Vorsprüngen gebildet sein, die in vier Löcher des Plattenkörpers passend einschnappen. Auch eine entsprechend geformte Metallklammer kann verwendet werden.

Eine bevorzugte Ausbildungsmöglichkeit der Zuhaltung gemäß Anspruch 8 ist die Verwendung einer Blattfeder, die auf der geschlossenen Seite der Klemmrille diese überquerend in dem Plattenkörper eingebettet und fixiert ist. Eine derartige Zuhaltung ist besonders vorteilhaft, weil sie keine Handhabungen des Anwenders verlangt. Sie ist bleibend in den Plattenkörper integriert und unterstützt die Rückstellfähigkeit des Polyurethan-Materials des Plattenkörpers zur Zusammenhaltung der Klemmrille bei eingelegtem Katheter. Die Blattfeder kann als gewölbte symmetrische Lamelle aus Metall ausgebildet sein, die in ihren beiden seitlichen Schenkeln Löcher aufweist, die sich mit Durchbrechungen in dem Plattenkörper decken oder in die Polyurethan-Material des Plattenkörpers eindringt, wodurch eine verbesserte Verankerung der Blattfeder in dem Plattenkörper verursacht wird. Die das Festnähen des Plattenkörpers in Gewebe oder auf der Haut ermöglichenden Löcher des Plattenkörpers dürfen von der Blattfeder nicht abgedeckt werden. Aus diesem Grunde muß entweder ihre Formgebung und/oder Abmessung entsprechend gewählt werden oder es müssen die Löcher in der Blattfeder sich mit den Durchbrechungen in dem Plattenkörper decken.

Vorteilhafte Weiterbildungen der Erfindung sind in den Unteransprüchen gekennzeichnet.

In der Zeichnung sind zwei Ausführungsbeispiele der Erfindung schematisch dargestellt.

Es zeigen:

Fig. 1 eine Draufsicht auf die Befestigungsvorrichtung mit eingelegtem Katheter,
Fig. 2 einen Längsschnitt durch den Plattenkörper längs der Linie II-II in Figur 1,
Fig. 3 einen Querschnitt durch den Plattenkörper nach Figur 1 längs der Linie III-III,
Fig. 4 eine schematische Seitenansicht der Ausgangsform der Blattfeder vor Umspritzung, und
Fign. 5, 6 und 7 den Figuren 1 bis 3 entsprechende Ansichten einer anderen Ausführungsform des Plattenkörpers mit Zuhaltung.

Die Vorrichtung zur Befestigung eines transcutanen oder implantierten Katheters 10, der ein Peridural-Katheter aus schlecht klebbarem Polyamid sein kann, besteht im wesentlichen aus einem weichflexiblen knopfförmigen Plattenkörper 30, vorzugsweise aus Polyurethan, der von einem Ende zum anderen von einer geraden Klemmrille 31 mit einem Axialschlitz 31a durchquert ist. Der Plattenkörper 30 ist in Draufsicht rechteckig mit runden Ecken und zu beiden Seiten der Klemmrille 31 weist er durchgehende Löcher 33 auf, die sich paarweise gegenüberliegen. Die Löcher 33 erlauben die Fixierung des Plattenkörpers 30 auf der Haut oder im Gewebe durch Ligatur.

Der Plattenkörper 30 ist auf der Seitenfläche 36, die den Axialschlitz 31a der Klemmrille 31 aufweist, leicht konkav gekrümmt und auf der gegenüberliegenden Seitenfläche 29, die den bündig abschließenden Grundsteg 31b der Klemmrille 31 enthält, im Querschnitt konvex gewölbt. Die Wölbungen erstrecken sich von einem Ende der Klemmrille 31 zum anderen. Gegen seine beiden länglichen Ränder, die etwa parallel zur Klemmrille 31 verlaufen, ist der Plattenkörper 30 abgeflacht.

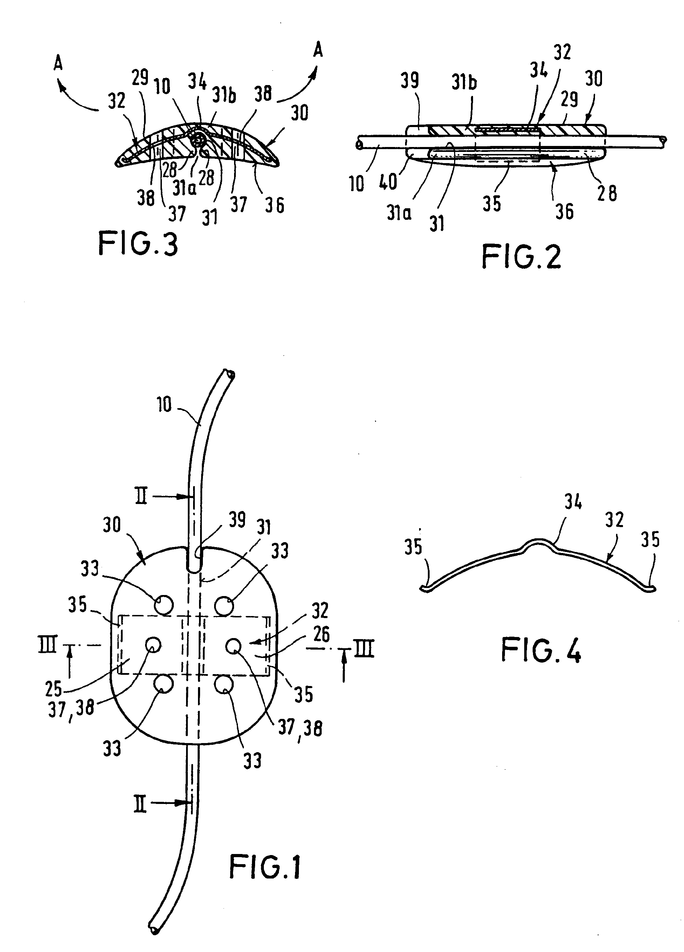

Bei dem Beispiel der Figuren 1 bis 4 hat die Klemmrille 31 kreisförmigen Querschnitt und sie erstreckt sich mit gleichem Durchmesser vom einen Ende zum anderen. Der Axialschlitz 31a wird von zwei beinah aneinanderstoßenden Lippen Seitenflanken 28 begrenzt, die bündig in der bei dem Beispiel unteren Seitenfläche 36 des Plattenkörpers 30 liegen. Um den Plattenkörper 30 zur Aufnahme des Katheters 10 in der Klemmrille 31 zu öffnen, wird er in Richtung der beiden Pfeile A (Fig. 3) gebogen und es wird der Katheter 10 quer in die Klemmrille 31 hineingedrückt. Der Durchmesser der Klemmrille 31 ist so bemessen, daß er etwas unter dem Durchmesser des zu fixierenden Katheters 10 liegt. Beim Zurückbiegen des Plattenkörpers 30 übt er einen leichten Druck auf den Katheter 10 aus, der ihn mit dem Plattenkörper 30 verbunden hält.

In dem Plattenkörper 30 aus weichflexiblem Kunststoffmaterial ist zur Steigerung der Klemmwirkung der Wände der Klemmrille 31 gegen einen eingelegten Katheter 10 eine Zuhaltung in Form einer Blattfeder 32 vorgesehen, die durch Umspritzung in den Plattenkörper 30 eingebettet ist. Die Blattfeder 32 aus Metall ist als gewölbte Lamelle gestaltet, die kürzer ist als der Plattenkörper 30 und sich im Mittelbereich des Plattenkörpers 30 quer zu der Klemmrille 31 erstreckt. Ihr zentraler Auswölbungsteil 34 verläuft durch den Grundsteg 31b des Plattenkörpers 30. Die beiden Enden 35 der Schenkel 25, 26 der Blattfeder 32 sind in die lippenartig verdünnten Seitenrandteile des Plattenkörpers 30 eingebettet, wie in Figuren 1 und 3 gezeigt ist. Sie sind vorteilhafterweise entgegen der Wölbung der Blattfeder 32 leicht gewölbt, damit sie keine Vorsprünge auf der glatten Unterfläche 36 des Plattenkörpers 30 hervorrufen und damit die rückfedernde Wirkung der Blattfeder 32 unterstützt wird. In den beiden Schenkeln 25, 26 der Blattfeder 32 ist je ein Loch 38 ausgebildet, das sich mit entsprechenden Durchbrechungen 37 (Figuren 1 und 3) in dem Plattenkörper 30 deckt und - wie die Löcher 33 - zum Festnähen des Plattenkörpers 30 auf der Haut oder im Gewebe dienen. Gegebenenfalls können diese Löcher 38 durch Aufnahme von Polyurethan-Material-Pfropfen bei der Umspritzung der Blattfeder 32 zur Verbesserung ihrer Verankerung in dem Plattenkörper 30 benutzt werden.

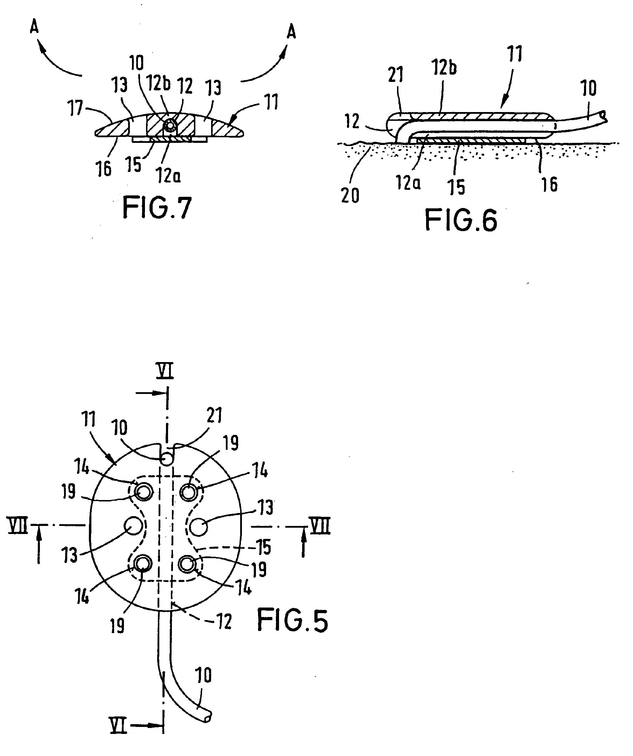

Um den aus der Haut oder aus Gewebe austretenden Katheter 10 gegen Knickung geschützt etwa rechtwinklig umgelenkt mit dem Plattenkörper 30 verbinden zu können (wie bei dem Beispiel der Figuren 5-7 angedeutet), hat dieser an einem Ende der Klemmrille 31 einen Durchlaß 39 in dem Grundsteg 31b der Klemmrille 31, der bei dem gezeichneten Beispiel als gegen das Ende des Plattenkörpers 30 offene Schlitzaussparung ausgebildet ist. Ein zweiter etwa deckungsgleicher Durchlaß 40 am gleichen Ende der Klemmrille 31 ist in den Lippen Seitenflanken 28 (Figur 2) ausgespart. Zur Schonung des Katheters 10 sind die Durchlässe 39,40 an der der Klemmrille 31 zugewandten Seite verrundet. Die gegen die beiden Seitenflächen 29 und 36 des Plattenkörpers 30 offenen Durchlässe 39,40 ermöglichen die rechtwinklige Umlenkung des Katheters 10 unabhängig von der Position des Plattenkörpers 30 in bezug auf seine Befestigungsunterlage, d.h. der Plattenkörper 30 kann - abweichend von der dargestellten Anordnung - auch so angebracht werden, daß der Axialschlitz 31a der Klemmrille 31 nach oben und ihr Grundsteg 31b nach unten weist.

Bei dem Beispiel nach Figuren 5, 6 und 7 ist ein Plattenkörper 11 ebenfalls aus weichflexiblem Kunststoffmaterial, vorzugsweise Polyurethan, hergestellt. Der Plattenkörper 11 ist in Draufsicht etwa oval und in Richtung seiner längeren Achse von einer geraden Klemmrille 12 mit einem Axialschlitz 12a durchsetzt. Der Axialschlitz 12a befindet sich an einer ebenen (in Figur 7 unteren) Seitenfläche 16. Die gegenüberliegende Seitenfläche 17 des Plattenkörpers 11 verläuft quer zur Klemmrille 12 konvex gewölbt, so daß die beiden länglichen Ränder des Plattenkörpers 11, die zur Klemmrille 12 parallel sind, durch verringerte Materialstärke erhöhte Flexibilität und Weichheit erhalten. Auf beiden Seiten der Klemmrille 12 weist der Plattenkörper 11 durchgehende Löcher 13 und 14 auf, die sich paarweise gegenüberliegen. Die Löcher 13 erlauben die Fixierung des Plattenkörpers 11 auf der Haut oder im Gewebe. Die beiden durch das Lochpaar 13 voneinander getrennten Lochpaare 14 dienen der Anbringung zweier Fadenligaturen zum Zusammenhalten der U-förmigen Klemmrille 12 in Querrichtung bei eingelegtem Katheter 10. Alternativ oder zusätzlich kann ein die Klemmrille 12 auf der Seitenfläche 16 überspannender Riegel 15 aus Kunststoff angebracht werden, der zur Freilassung der beiden Löcher 13 des Plattenkörpers 11 Hyperbelform haben kann (Figur 5). Der Riegel 15 ist eine Flachplatte, die auf einer Seite vier Vorsprünge 19 trägt, welche in die Löcher 14 des Plattenkörpers 11 druckknopfartig eingeschnappt oder im Preßsitz festgeklemmt sind. Einen zusätzlichen Halt für den in die Klemmrille 12 eingelegten Katheter 10 bietet die Oberflächenklebrigkeit des Polyurethan-Materials des Plattenkörpers 11, weil die Wände der U-förmigen Klemmrille 12 gummielastisch gegen den etwas größer als der Klemmrillendurchmesser bemessenen Katheter 10 andrücken. Um vor Anbringung des Riegels 15 den Katheter 10 in die Klemmrille 12 einzulegen, wird der Plattenkörper 11 in Richtung der beiden Pfeile A (Figur 7) um den Grundsteg 12b der Klemmrille 12 gebogen, so daß die Klemmrille 12 aufgeweitet wird. Die eigene Elastizität des Plattenkörper-Materials sorgt für eine Rückstellung des Plattenkörpers in die Grundposition, in der die Klemmwirkung der Klemmrille 12 erzielt und der Riegel 15 angebracht werden kann.

Auch der Plattenkörper 11 kann wie in Figur 6 dargestellt oder in umgekehrter Position auf der Haut 20 befestigt werden. Bei umgekehrter Anbringung dient ein Durchlaß 21 in Form einer offenen Schlitzaussparung in dem Grundsteg 12b an einem Ende der Klemmrille 12 als Knickschutz für den Katheter 10. Zwischen dem abgebogenen Katheter 10 und dem Durchlaß 21 ist eine gewisse geringfügige Klemmwirkung vorhanden. Zur Schonung des Katheters 10 ist der Durchlaß 21 an der der Klemmrille 12 zugewandten Seite verrundet.

**Patentansprüche**

1. Vorrichtung zur Befestigung eines transcutanen oder implantierten Katheters (10), bestehend aus einer Platte aus biegbarem Material, die mit einer endseitig offenen, axial geschlitzten Klemmrille mit federelastischen Seitenflanken für den Katheter (10) versehen ist, **dadurch gekennzeichnet,** daß die Platte einen von zwei vorsprungslosen Seitenflächen begrenzten weichflexiblen, knopfförmigen Plattenkörper (11;30) aufweist, in den die Klemmrille (12;31) eingelassen ist.

2. Vorrichtung nach Anspruch 1, dadurch gekennzeichnet, daß in dem Plattenkörper (11;30) auf beiden Längsseiten der Klemmrille (12;31) durchgehende Löcher (13,14;33) vorgesehen sind.

3. Vorrichtung nach Anspruch 1 oder 2, dadurch gekennzeichnet, daß der Plattenkörper (11;30) in dem die gerade Klemmrille (12;31) aufweisenden Bereich verdickt ausgebildet ist und daß die dem Axialschlitz (12a;31a) der Klemmrille (12;31) abgewandte Seitenfläche (17;29) konvex gewölbt ist.

4. Vorrichtung nach einem der Ansprüche 1 bis 3, dadurch gekennzeichnet, daß die Klemmrille (31) im Querschnitt im wesentlichen kreisförmig ist und daß an wenigstens einem Ende der Klemmrille (31) wenigstens ein zu der Dicke des Plattenkörpers (11;30) quergerichteter Durchlaß (39;40) für den abgewinkelten Katheter (10) ausgebildet ist.

5. Vorrichtung nach einem der Ansprüche 1 bis 3, dadurch gekennzeichnet, daß die Klemmrille (12) im Querschnitt im wesentlichen U-förmig ist und daß an wenigstens einem Ende der Klemmrille (12) ein zu der Dicke des Plattenkörpers (11) quergerichteter Durchlaß (21) für den abgewinkelten Katheter (10) ausgebildet ist.

6. Vorrichtung nach einem der Ansprüche 1 bis 5, dadurch gekennzeichnet, daß der Plattenkörper (11;30) eine Zuhaltung für den Axialschlitz (12a;31a) der Klemmrille (12;31) aufweist.

7. Vorrichtung nach Anspruch 6, dadurch gekennzeichnet, daß die Zuhaltung aus einem den Axialschlitz (12a) der Klemmrille (12) überspannenden Riegel (15) mit Vorsprüngen (19) besteht, die in einige der Löcher (14) des Plattenkörpers (11) eingeschnappt sind.

8. Vorrichtung nach Anspruch 6, dadurch gekennzeichnet, daß die Zuhaltung aus einer Blattfeder (32) besteht, die auf der geschlossenen Seite der Klemmrille (31) diese überquerend in dem Plattenkörper (30) eingebettet und fixiert ist.

9. Vorrichtung nach Anspruch 8, dadurch gekennzeichnet, daß die Blattfeder (32) als gewölbte, symmetrische Lamelle aus Metall ausgebildet ist, die in ihren beiden Schenkeln (25,26) Löcher (38) aufweist, die sich mit Durchbrechungen (37) in dem Plattenkörper (30) decken.

10. Vorrichtung nach Anspruch 9, dadurch gekennzeichnet, daß die Enden (35) der Schenkel (25,26) der Blattfeder (32) jeweils in einem ihrer Wölbung entgegengesetzten Bogen auslaufen.

11. Vorrichtung nach einem der Ansprüche 1 bis 10, dadurch gekennzeichnet, daß der Plattenkörper (11;30) aus Polyurethan hergestellt ist.

**Claims**

1. Device for fastening a transcutaneous or an implanted catheter (10), comprising a plate of bendable material provided with an axially slotted clamping groove for a catheter (10), which is open at the ends and has resilient lateral flanks, **characterized in that** the plate is provided with a soft flexible, button-shaped plate body (11;30) that is defined by two lateral faces without projections, in which plate body the clamping groove (12;31) is recessed.

2. Device according to claim 1, characterized in that the plate body (11;30) is provided with through-going holes (13,14;33) on both longitudinal sides of the clamping groove (12;31).

3. Device according to claim 1 or 2, characterized in that the plate body (11;30) is enlarged in the region containing the straight clamping groove (12;31) and that the lateral face (17;29) facing away from the axial slot (12a;31a) of the clamping groove (12;31) is convexly curved.

4. Device according to one of claims 1 to 3, characterized in that the clamping groove (31) has a substantially circular cross section and that at least one end of the clamping groove (31) is provided with at least one passage (39;40) for the bent catheter (10), which passage extends transversely to the thickness of the plate body (11;30).

5. Device according to one of claims 1 to 3, characterized in that the clamping groove (12) has a substantially U-shaped cross section and that at least one end of the clamping groove (12) is provided with a passage (21) for the bent catheter (10), which passage extends transversely to the thickness of the plate body (11).

6. Device according to one of claims 1 to 5, characterized in that the plate body (11;30) is provided with a locking device for the axial slot (12a;31a) of the clamping groove (12;31).

7. Device according to claim 6, characterized in that the locking device consists of a tie (15) with projections (19) snapped into some of the holes (14) of the plate body (11), which tie bridges the axial slot (12a) of the clamping groove (12).

8. Device according to claim 6, characterized in that the locking device consists of a leaf spring (32) that is embedded and fixed in the plate body (30) on the closed side of the clamping groove (31) which it bridges.

9. Device according to claim 8, characterized in that the leaf spring (32) is shaped as a curved, symmetric metal lamella having its two legs (25,26) provided with holes (38) that coincide with passages (37) in the plate body (30).

10. Device according to claim 9, characterized in that the ends (35) of said legs (25,26) of said leaf spring (32) each end in a bow opposite to their respective curve.

11. Device according to one of claims 1 to 10, characterized in that the plate body (11;30) is made of polyurethane.


## Revendications

1. Dispositif pour la fixation d'un cathéter (10) transcutané ou implanté, le dispositif se composant d'une plaque en un matériau flexible qui est pourvue d'une rainure de pincement ouverte aux extrémités, axialement fendue et comportant des flancs latéraux élastiques pour le cathéter (10),
**caractérisé en ce que** la plaque présente un corps de plaque souple (11; 30), en forme de bouton, limité par deux faces latérales libres de toute protubérance, et dans lequel est formée la rainure de pincement (12; 31).

2. Dispositif selon la revendication 1, caractérisé en ce que dans le corps de plaque (11; 30) sont prévus des trous traversants (13, 14; 33), de chaque côté longitudinal de la rainure de pincement (12; 31).

3. Dispositif selon la revendication 1 ou 2, caractérisé en ce que le corps de plaque (11; 30) est d'une épaisseur plus importante dans la zone présentant la rainure de pincement rectiligne ( 12; 31 ), et en ce que la face latérale (17; 29) opposée à celle où se trouve la rainure de pincement ( 12; 31 ), est bombée de manière convexe.

4. Dispositif selon l'une des revendications 1 à 3, caractérisé en ce que la rainure de pincement (31) présente en section droite, une forme essentiellement circulaire, et en ce qu'à au moins une extrémité de la rainure de pincement (31), est formé au moins un passage (39, 40) orienté transversalement à l'épaisseur du corps de plaque (11; 30) et destiné au cathéter coudé (10).

5. Dispositif selon l'une des revendications 1 à 3, caractérisé en ce que la rainure de pincement (12) présente en section droite, une forme essentiellement en "U", et en ce qu'a au moins une extrémité de la rainure de pincement (12), est formé au moins un passage (21) orienté transversalement à l'épaisseur du corps de plaque (11) et destiné au cathéter coudé (10).

6. Dispositif selon l'une des revendications 1 à 5, caractérisé en ce que le corps de plaque (11; 30) présente un système de verrouillage pour la fente axiale (12a; 31a) de la rainure de pincement (12; 31).

7. Dispositif selon la revendication 6, caractérisé en ce que le système de verrouillage est constitué par un verrou (15) qui ferme la fente axiale (12a) de la rainure de pincement (12) en passant pardessus d'elle, et qui comporte des protubérances (19) qui sont encliquetées dans certains des trous (14) du corps de plaque (11).

8. Dispositif selon la revendication 6, caractérisé en ce que le système de verrouillage est constitué par un ressort à lame (32) qui est noyé et fixé dans le corps de plaque (30) sur le côté fermé de la rainure de pincement (31) en passant transversalement au-dessus de celle-ci.

9. Dispositif selon la revendication 8, caractérisé en ce que le ressort à lame (32) est réalisé sous la forme d'une lamelle bombée symétrique en métal, présentant dans ses deux branches latérales (25, 26), des trous (38) qui coïncident avec des passages (37) dans le corps de plaque (30).

10. Dispositif selon la revendication 9, caractérisé en ce que les extrémités (35) des branches (25, 26) du ressort à lame (32) se terminent chacune par une partie cintrée s'étendant dans la direction opposée à sa courbure.

11. Dispositif selon l'une des revendication 1 à 10, caractérisé en ce que le corps de plaque (11; 30) est fabriqué en polyuréthanne.

FIG.3

FIG.2

FIG.1

FIG.4

FIG.7

FIG.6

FIG.5